(19) Europäisches Patentamt / European Patent Office / Office européen des brevets

(11) **EP 4 429 648 B1**

(12) **FASCICULE DE BREVET EUROPEEN**

(45) Date de publication et mention de la délivrance du brevet:
**13.05.2026 Bulletin 2026/20**

(21) Numéro de dépôt: **22817139.3**

(22) Date de dépôt: **08.11.2022**

(51) Classification Internationale des Brevets (IPC):
*A61K 31/13* (2006.01)   *A61K 31/27* (2006.01)
*A61K 31/445* (2006.01)   *A61K 31/473* (2006.01)
*A61K 31/55* (2006.01)   *A61P 25/00* (2006.01)
*A61P 25/08* (2006.01)   *A61P 25/16* (2006.01)
*A61P 25/28* (2006.01)   *A61K 45/06* (2006.01)

(52) Classification Coopérative des Brevets (CPC):
(C-Sets disponibles)
A61K 31/13; A61K 31/27; A61K 31/445;
A61K 31/473; A61K 31/55; A61K 45/06;
A61P 25/00; A61P 25/08; A61P 25/16; A61P 25/28
(Cont.)

(86) Numéro de dépôt international:
**PCT/EP2022/081184**

(87) Numéro de publication internationale:
**WO 2023/079187 (11.05.2023 Gazette 2023/19)**

(54) **NOUVELLES COMBINAISONS SYNERGIQUES A BASE DE FLUOROÉTHYLNORMÉMANTINE (FENM) ET UN INHIBITEUR DE L'ACÉTYLCHOLINESTÉRASE POUR SON UTILISATION DANS LE TRATEMENT DES MALADIES NÉURODÉGÉRATIVES**

NEUE SYNERGISCHE KOMBINATIONEN AUF DER BASIS VON FLUORETHYLNOREMMANTIN (FENM) UND EINEM ACETYLCHOLINESTERASEHEMMER ZUR VERWENDUNG BEI DER BEHANDLUNG NEURODEGEATIVER ERKRANKUNGEN

NOVEL SYNERGISTIC COMBINATIONS BASED ON FLUOROETHYLNOREMMANTINE (FENM) AND AN ACETYLCHOLINESTERASE INHIBITOR FOR USE IN THE TREATMENT OF NEURODEGEATIVE DISEASES

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **08.11.2021 FR 2111841**

(43) Date de publication de la demande:
**18.09.2024 Bulletin 2024/38**

(73) Titulaires:
• **REST THERAPEUTICS**
  **75006 Paris (FR)**
• **INSTITUT NATIONAL DE LA SANTE ET DE LA RECHERCHE MEDICALE - INSERM**
  **75013 Paris (FR)**
• **UNIVERSITE DE MONTPELLIER**
  **34090 Montpellier (FR)**

• **Ecole Pratique des Hautes Etudes**
  **75014 Paris (FR)**

(72) Inventeurs:
• **RUBINSTENN, Gilles**
  **75005 PARIS (FR)**
• **MAURICE, Tangui**
  **34980 SAINT-GELY-DU-FESC (FR)**
• **FREYSSIN, Aline**
  **34230 ADISSAN (FR)**

(74) Mandataire: **A.P.I. Conseil**
  **Technopôle Hélioparc**
  **4, rue Jules Ferry**
  **64000 Pau (FR)**

(56) Documents cités:
  **WO-A1-2014/191424   WO-A1-2020/232246**

- **PATEL LAXESHKUMAR ET AL: "Combination Therapy for Alzheimer's Disease", DRUGS & AGING, ADIS INTERNATIONAL LTD, NZ, vol. 28, no. 7, 1 January 2011 (2011-01-01), pages 539 - 546, XP008163122, ISSN: 1170-229X**
- **S. COULY ET AL: "Anti-amnesic and neuroprotective effects of Fluoroethylnormemantine (FENM) in a pharmacological mouse model of Alzheimer's disease", INT. J. NEUROPSYCHOPHARMACOLOGY, 25 September 2020 (2020-09-25), pages 1 - 16, XP055764703, Retrieved from the Internet <URL:https://watermark.silverchair.com/ pyaa075.pdf? token=AQECAHi208BE49Ooan9kkhW_Er cy7Dm3ZL_9Cf3qfKAc485ysgAAAs0wggLJBgkq hkiG9w0BBwagggK6MIlCtglBADCCAq8GCSqGS Ib3DQEHATAeBglghkgBZQMEAS4wEQQM7dPB p4EGAa6g-uIbAgEQgllCgILuJRaAZoOfeal1lETP mf5vg-8Vjqwp8PtDqT48ZA5hkRxs BiO6l5ChSJVcN7JQQbc380Gs2ryQdpKgEie5 k_0u7Eib> [retrieved on 20210113]**

Remarques:

Le document complet y compris le(s) table(s) de référence et le(s) listage(s) de séquence sont téléchargeables sur le site internet de l'OEB

(52) Classification Coopérative des Brevets (CPC): (Cont.)

C-Sets
**A61K 31/13, A61K 2300/00;
A61K 31/27, A61K 2300/00;
A61K 31/445, A61K 2300/00;
A61K 31/473, A61K 2300/00;
A61K 31/55, A61K 2300/00**

## Description

L'invention s'intéresse au domaine des maladies neurodégénératives.

L'invention se rapporte plus particulièrement à des compositions comprenant une combinaison synergique de 3(2-Fluoroéthyl)tricyclo [3.3.1.13,7]decan-1-amine (Fluoroéthylnormémantine, FENM) avec au moins un inhibiteur d'acétylcholinestérase.

L'invention se rapporte également à ces combinaisons ou des compositions les comprenant pour leur utilisation dans le traitement de pathologies neurodégénératives et, plus particulièrement, dans la prévention et/ou le traitement des troubles cognitifs de ces pathologies.

## Art antérieur

L'Organisation Mondiale de la Santé (OMS) estime que d'ici 2050 le nombre de personnes âgées de plus de 60 ans devrait atteindre deux milliards. Ce vieillissement sans précédent de la population mondiale laisse entrevoir une forte pression des maladies chroniques liées au vieillissement sur les systèmes de santé. La démence est une de celles-ci. Ainsi, l'OMS estime que le nombre total de personnes atteintes de démence devrait dépasser 150 millions de personnes à l'horizon de 2050.

La démence se caractérise par une dégradation des fonctions cognitives notamment de la mémoire et du raisonnement qui impacte le comportement du malade et sa capacité à effectuer des tâches pourtant quotidiennes. La démence est un syndrome qui recouvre des pathologies aux étiologies très diverses qui affectent des zones différentes du cerveau et/ou d'autres régions du système nerveux central et impliquent notamment la neurodégénérescence et la mort des cellules neuronales. En lien direct avec le vieillissement, la dysfonction mitochondriale et le stress oxydatif jouent un rôle crucial dans la pathogenèse des maladies neurodégénératives. Ces pathologies et syndromes sont également souvent en lien avec une accumulation anormale de certaines protéines et/ou l'accumulation de protéines mutées et/ou anormalement repliées telle qu'observée dans les amyloïdoses à $A\beta$, les tauopathies, les synucléinopathies, l'agrégation de la superoxyde dismutase-1 (SOD1), de la polyglutamine, de la protéine TDP-43.

La maladie d'Alzheimer est la cause la plus courante de démence et serait à l'origine de 60-70% des cas (source OMS).

Aujourd'hui, plusieurs molécules sont autorisées dans le traitement symptomatique de la maladie d'Alzheimer. Ce sont les anticholinestérasiques tels que le donépézil, la rivastigmine, ou la galantamine qui bénéficient d'autorisations de mise sur le marché, en monothérapie, au stade léger, modéré ou modérément sévère de la maladie. La mémantine, un antagoniste voltage-dépendant non compétitif des récepteurs NMDA, est autorisée aux stades modéré et sévère de la maladie et n'est pas autorisée au stade léger de la maladie. Ces composés ne sont pas conseillés au stade précoce de la maladie, de part un manque d'efficacité clinique. De plus, leurs effets sont symptomatiques et limités et n'ont été mis en évidence qu'à court terme (en moyenne 6 mois) chez près des deux tiers des patients inclus dans les études cliniques (source, Haute Autorité de Santé, France). Un traitement combinatoire de donépézil et de mémantine, sous le nom de marque Acrescent® a vu son autorisation de mise sur le marché (AMM) refusée par l'Agence Européenne des Médicaments (EMA) devant les faibles bénéfices de cette combinaison (20 mg mémantine/10 mg donépézil) au regard des composés utilisés seuls (EMA, 18, octobre 2012). Le seul intérêt se limiterait à la prise unique d'un cachet en remplacement de deux, chez des patients sujets à des troubles cognitifs ; ce traitement a malgré tout été autorisé aux États-Unis par la FDA. En outre, la supposée efficacité des anticholinestérasiques au stade précoce de la maladie repose sur une seule étude réalisée spécifiquement sur des patients à ce stade au cours de laquelle un bénéfice modeste a été observé chez les seuls patients qui toléraient des doses de 10 mg par jour de donépézil. Les autorités de santé, en France, considèrent que cette étude ne permet pas de conclure à un intérêt de commencer ce traitement au stade léger de la maladie (Source Haute Autorité de Santé, France, 2012).

Les inhibiteurs d'acétylcholinestérase sont également associés à un risque d'effets indésirables graves ou susceptibles d'altérer la qualité de vie et/ou de nécessiter l'arrêt du traitement. Il peut s'agir de troubles digestifs (diarrhée, vomissements), de troubles cardiovasculaires (bradycardie, syncopes), voire de troubles neuropsychiatriques (vertiges, confusion mentale) contreproductifs dans le cas de traitement de la démence.

Le risque d'effets indésirables qui augmente avec la durée du traitement, outre l'absence de bénéfice au-delà de six mois, préempte fortement la possibilité de leur utilisation sur le long court. En pratique, ces traitements sont appliqués aux patients aux capacités cognitives déjà dégradées, au stade modéré, voire avancé, de la maladie.

La demande WO 2014/191424 décrit la FENM marquée au $^{18}$F pour le marquage des récepteurs NMDA et leur visualisation par tomographie par émission de positons pour étudier la distribution de ces récepteurs et leur réaction à des traitements médicamenteux. La demande WO 2019/115833 décrit la FENM dans le traitement de troubles liés à l'anxiété et à la dépression.

La demande WO 2013/064579 décrit une combinaison d'agent bloquant les connexines (tel que l'acide méclofénamique) avec un inhibiteur de l'acétylcholine estérase (tel que le donépézil) pour leur utilisation dans le traitement de troubles cognitifs.

L'aducanumab est un anticorps monoclonal anti-amyloïde dont l'utilisation vise à diminuer la charge amyloïde dans le cerveau. L'aducanumab a bénéficié d'une AMM délivrée par la FDA en 2021, pour le traitement de la maladie d'Alzheimer au stade précoce ou prodromal avec présence avérée de dépôts amyloïdes. Devant des résultats non reproduits concernant un moindre déclin cognitif chez les patients traités, et tenant compte du fait de l'absence de solution thérapeutiques, l'AMM a été conditionnée à une réévaluation du produit pour confirmer son intérêt clinique (<www.fda.gov>). Environ 35% des patients traités ont présenté des microhémorragies cérébrales et des oedèmes cérébraux (source France Alzheimer, <www.francealzheimer.org>). Le coût du traitement, qui requiert des administrations intraveineuses mensuelles, est estimé à 56 000 dollars par an.

Le diagnostic d'une démence ou de la maladie d'Alzheimer à un stade précoce induit une demande importante de traitement de la part du patient et de son entourage, en vue de stopper ou de ralentir le plus tôt possible l'évolution de la maladie. Ce besoin est aujourd'hui insatisfait. Également, il existe un besoin important pour des traitements plus efficaces tant sur les symptômes traités que sur la durée d'efficacité du traitement chez les patients au stade plus avancé de la maladie.

Problème technique

L'invention a pour but de remédier aux inconvénients de l'art antérieur. En particulier, l'invention a pour but de proposer une nouvelle thérapie aux atteintes cognitives dans les pathologies neurodégénératives et notamment la maladie d'Alzheimer (MA). Cette nouvelle thérapie est basée sur l'action combinée de la fluoroéthylnormémantine (FENM) et d'au moins un inhibiteur de l'acétylcholinestérase. La synergie d'action identifiée entre ces molécules permet d'une part d'obtenir des effets particulièrement efficaces dans la préservation des capacités cognitives et/ou de diminuer les doses utilisées pour ces composés. Cette diminution permet de diminuer le risque d'effets indésirables voire d'augmenter la durée possible du traitement tout en obtenant une amélioration supérieure pour des doses particulièrement faibles. Cette efficacité améliorée permet d'envisager l'utilisation de ce traitement, y compris au stade précoce de la maladie.

Brève description de l'invention

Les demandeurs ont découvert de manière tout à fait surprenante que l'association des effets de la FENM avec ceux des inhibiteurs de l'acétylcholinestérase a un effet synergique dans le traitement des symptômes cognitifs dans un modèle de souris intoxiquée par administration intracérébrale d'oligomères de peptide amyloïde $\beta_{25-35}$ ($A\beta_{25-35}$). Cette action synergique est observée dans des tests cognitifs de mémoire de travail à court terme, mais également de mémoire contextuelle à long terme. De tels effets synergiques permettent d'envisager des traitements plus efficaces que ceux actuellement appliqués et d'élargir la population de patients éligibles à ces traitements par rapport aux pratiques actuelles.

En conséquence, un objet de la présente invention concerne une composition comprenant une combinaison synergique de 3-(2-fluoroéthyl)adamantan-1-amine (FENM) ou l'un quelconque de ses sels pharmaceutiquement acceptable et d'au moins un inhibiteur de l'acétylcholinestérase ou l'un quelconque de ses sels pharmaceutiquement acceptable.

Dans un mode de réalisation particulier, ledit au moins un inhibiteur de l'acétylcholine estérase est sélectionné parmi le donépézil, la galantamine, la rivastigmine, la tacrine, ou l'un quelconque de leurs sels pharmaceutiquement acceptable. En effet, ces molécules bénéficient d'une AMM ou ont déjà été testées chez l'homme et leurs propriétés pharmacocinétiques et pharmacodynamiques sont déjà connues, ce qui est un avantage particulier.

Selon d'autres caractéristiques de la composition selon l'invention :

- les composés sont formulés ensemble ou séparément.

- la FENM et/ou ledit au moins un inhibiteur d'acétylcholinestérase est mélangé avec un excipient pharmaceutiquement acceptable.

L'interaction synergique entre la FENM et avec les inhibiteurs de l'acétylcholinestérase découverte par les inventeurs permet d'envisager, comparé aux monothérapies, une meilleure prise en charge thérapeutique des patients, avec un gain d'efficacité du traitement et/ou une diminution des doses administrées et donc une diminution des possibles effets secondaires associés au composés actifs de la composition de l'invention à savoir la FENM et l'au moins un inhibiteur de l'acétylcholinestérase. Néanmoins, à ces faibles doses, le bénéfice thérapeutique est sauvegardé voire amélioré.

Ainsi, dans des modes de réalisation particuliers et indépendants, dans la composition selon l'invention :

- la dose de FENM est compatible avec un dosage de FENM inférieur ou égal à 20 mg/jour,
- ledit au moins un inhibiteur d'acétylcholinestérase est le donépézil qui est présent à une dose compatible avec un dosage inférieur ou égal à 10 mg/jour,
- ledit au moins un inhibiteur d'acétylcholinestérase est la rivastigmine qui est présente à une dose compatible avec un dosage inférieur ou égal à 3 mg/jour, et/ou
- ledit au moins un inhibiteur d'acétylcholinestérase est la galantamine qui est présente à une dose compatible avec un dosage inférieur ou égal à 16 mg/jour.

Dans un autre de ses modes de réalisation particuliers, dans la composition selon l'invention, le ratio molaire FENM/au moins un inhibiteur d'acétylcholine estérase est inférieur ou égal à 4, inférieur ou égal à 3, de préférence inférieur ou égal à 2, de préférence inférieur ou égal à 1.

Un objet particulier de la présente invention concerne la composition telle que décrite ci-dessus, dans l'un quelconque de ses modes de réalisation, pour son utilisation en tant que médicament.

Plus particulièrement, et comme démontré dans la partie expérimentale, la composition comprenant une combinaison synergique de FENM et d'au moins un inhibiteur de l'acétylcholinestérase est particulièrement efficace pour contrer les atteintes cognitives dans un modèle de pathologie neurodégénérative. Ainsi un autre objet de l'invention concerne ladite composition dans l'un quelconque de ses modes de réalisation décrits ci-dessus pour son utilisation dans le traitement d'une pathologie sélectionnée parmi les tauopathies, synucléinopathies, les amyloïdopathies, la maladie d'Alzheimer, la maladie de Parkinson, l'atrophie multisystématisée, la maladie de Huntington, l'atrophie corticale postérieure, la maladie de Pick, l'épilepsie, la démence vasculaire, la démence fronto-temporale, la démence à corps de Lewy, la sclérose latérale amyotrophique.

Dans la composition de l'invention, aux fins de l'une quelconque des utilisations décrites précédemment, la FENM et de l'au moins un inhibiteur l'acétylcholinestérase sont administrés séparément, concomitamment ou séquentiellement. Par exemple, cela peut être opéré par l'utilisation de formulations spécifiques de la FENM d'une part et de l'au moins un inhibiteur l'acétylcholinestérase d'autre part entrainant une cinétique d'infusion différenciée pour chacun des composés inclus dans une composition unitaire ; alternativement selon un autre exemple, les au moins FENM d'une part et de l'au moins un inhibiteur l'acétylcholinestérase de la combinaison d'autre part sont formulés et administrés dans des formes galéniques individualisées.

Un autre objet de l'invention réside dans la FENM, ou un sel pharmaceutiquement acceptable de celle-ci, en combinaison synergique avec au moins au moins un inhibiteur de l'acétylcholinestérase sélectionné parmi le donépézil, la galantamine, la rivastigmine, la tacrine ou l'un quelconque de leurs sels pharmaceutiquement acceptable, pour une utilisation dans le traitement d'une pathologie sélectionnée parmi les tauopathies, synucléinopathies, les amyloïdopathies, la maladie d'Alzheimer, la maladie de Parkinson, l'atrophie multisystématisée, la maladie de Huntington, l'atrophie corticale postérieure, la maladie de Pick, l'épilepsie, la démence vasculaire, la démence fronto-temporale, la démence à corps de Lewy, la sclérose latérale amyotrophique.

**Figures**

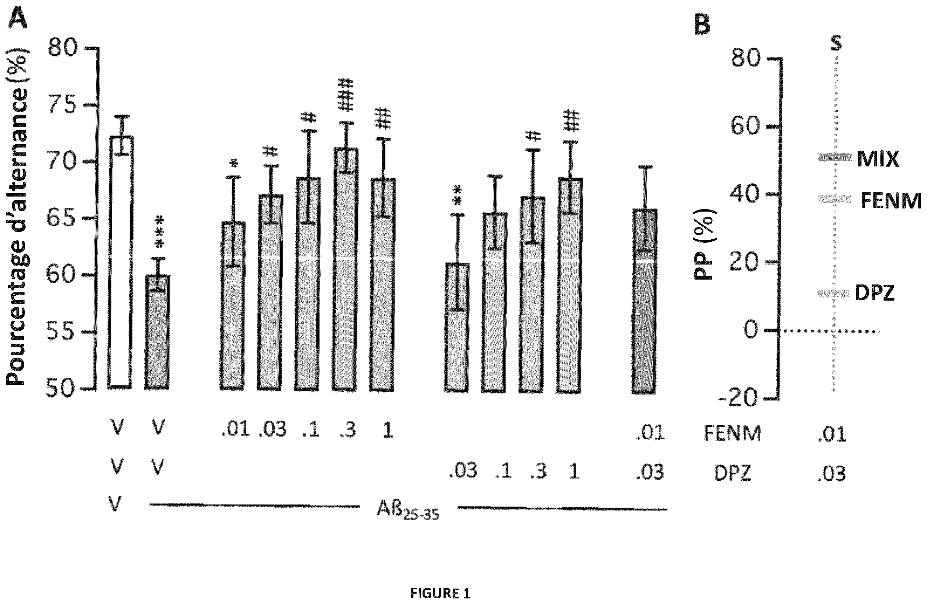

Fig 1 **(A)** effet symptomatique de la FENM, du donépézil (DPZ) ou de la combinaison FENM-DPZ sur l'altération de la mémoire induite par le peptide $A\beta_{25-35}$ oligomérisé dans le test du labyrinthe en Y; Données $\pm$ erreur type. ANOVA: $F_{(14,200)} = 2.511$, $p = 0.0026$, n = 30-31 par groupe pour les contrôles traités au véhicule et non intoxiqués et 11-20 pour les groupes traités. **°$p$<°0.01, *** $p < 0.001$ vs. traités au véhicule et non intoxiqués ; #$p < 0.05$, ##$p < 0.01$, ###°$p$°< 0.001 *vs.* Intoxiqués avec les oligomères $A\beta_{25-35}$ et traités au véhicule ; Dunnett's test. (B) Echelle de protection montrant le niveau de protection (PP) conféré par le donépézil (DPZ), la FENM ou le mix FENM-Donépézil (MIX). L'indice de combinaison calculé est inférieur à 1. Les 100% correspondent au niveau de performance des souris non intoxiquées par les oligomères $A\beta_{25-35}$ et traitées au véhicule. 0% de protection correspond au niveau de performance des souris intoxiquées et traitées au véhicule. S: effet synergique ; V : véhicule.

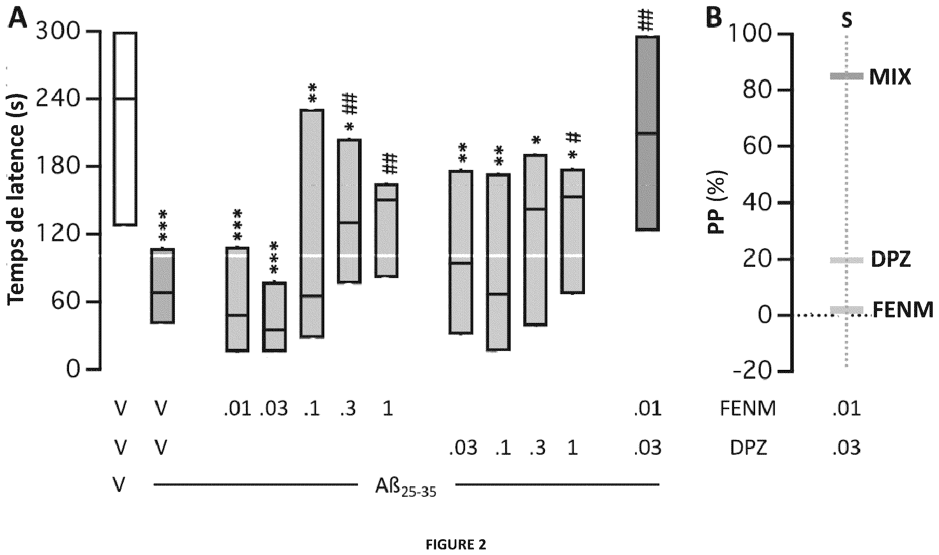

Fig 2 **(A)** effet symptomatique de la FENM du donépézil (DPZ) ou de la combinaison FENM-DPZ sur l'altération de la mémoire induite par l'intoxication au peptide $A\beta_{25-35}$ oligomérisé dans le test de l'évitement passif. Les résultats sont des données non paramétriques et sont présentés en médiane et écarts 25%-75%. L'entrainement a lieu au jour 9, 48h après le dernier traitement. La rétention a été testée le jour 10, 24 heures après l'entrainement. Kruskal-Wallis ANOVA: $H°=°54.27$, $p < 0.0001$, n = 34-36 par groupe pour les animaux contrôles non intoxiqués et traités au véhicule et 11-20 pour les groupes intoxiqués aux oligomères $A\beta_{25-35}$ et traités avec les composés. * $p < 0.05$, ** $p < 0.01$, ***$p < 0.001$ vs. groupe non intoxiqué et traité au véhicule ; # $p < 0.05$, ## $p < 0.01$, ### $p < 0.001$ vs. groupe intoxiqué à $A\beta_{25-35}$ et traité au véhicule ; test de Dunn. **(B)** Echelle de protection montrant le niveau de protection (PP) conféré par le donépézil (DPZ) la FENM ou le mix FENM-donépézil (MIX). L'indice de combinaison calculé est inférieur à 1. Les 100% correspondent au niveau de performance des souris non intoxiquées par les oligomères $A\beta_{25-35}$ et traitées au véhicule. 0% de protection correspond au niveau de performance des souris intoxiquées et traitées au véhicule. S: effet synergique ; V : véhicule.

## Description de l'invention

Définitions

Dans le contexte de la présente invention, la mention d'un médicament ou d'un composé spécifique inclut non seulement le médicament ou le composé spécifiquement nommé, mais également tout sel, hydrate, dérivé, isomère, racémate, composition énantiomériquement pure, conjugué ou prodrogue correspondante, pharmaceutiquement acceptable, de la molécule active du médicament ou dudit composé. De manière préférée, la mention d'un composé inclut le composé spécifiquement nommé, ainsi que tout sel, hydrate, isomère, racémate, isomère, composition énantiomériquement pure, pharmaceutiquement acceptable dudit composé. De manière plus préférée, la désignation d'un composé est destinée à désigner le composé comme spécifiquement désigné en soi, ainsi que tout sel pharmaceutiquement acceptable de celui-ci. Sauf mention contraire néanmoins, la mention en unité de masse ou en unité de masse par jour de la quantité d'un composé de la composition ou la combinaison selon l'invention, s'entend du composé désigné en soi.

Par « sels pharmaceutiquement acceptables », on entend au sens de l'invention, un sel d'addition d'acide inorganique ou organique pharmaceutiquement acceptable et relativement non toxique d'un composé de la présente invention. La formation de sel pharmaceutique consiste à coupler une molécule de médicament acide, basique ou zwitterionique avec un contre-ion pour créer une version saline du médicament. Une grande variété d'espèces chimiques peut être utilisée dans la réaction de neutralisation. Les sels pharmaceutiquement acceptables de l'invention comprennent donc ceux obtenus en faisant réagir le composé concerné, quand il fonctionne comme une base, avec un acide inorganique ou organique pour former un sel, par exemple, des sels d'acide acétique, d'acide nitrique, d'acide tartrique, d'acide chlorhydrique, acide bromhydrique, acide sulfurique, acide phosphorique, acide méthane sulfonique, acide camphre sulfonique, acide oxalique, acide maléique, acide succinique ou acide citrique. Les sels pharmaceutiquement acceptables de l'invention comprennent également ceux dans lesquels, quand le composé concerné fonctionne comme un acide, ledit composé est mis à réagir avec une base appropriée pour former, par exemple, des sels de sodium, de potassium, de calcium, de magnésium, d'ammonium ou de choline. Bien que la plupart des sels d'un principe actif donné soient bioéquivalents, certains peuvent avoir, entre autres, des propriétés de solubilité ou de biodisponibilité accrues. La sélection du sel est maintenant une opération standard courante dans le processus de développement de médicaments comme enseigné par Stahl et Wermuth dans leur manuel (Stahl and Wermuth).

Des exemples des composés utilisables pour mettre en œuvre une combinaison de la FENM avec des inhibiteurs de l'acétylcholinestérase selon l'invention sont listés dans le tableau 1 ci-dessous.

Tableau 1

| Composé | Numéro de référence *CAS*[†] |
|---|---|
| FENM | 1639210-26-6 |
| FENM HBr | NA |
| FNEM HCl | NA |
| donépézil | 120014-06-4 ; 120011-70-3 |
| galantamine | 357-70-0 ; 1953-04-4 ; 5072-47-9 |
| rivastigmine | 123441-03-2 ; 129101-54-8 |
| tacrine | 321-64-2 ; 1684-40-8 ; 7149-50-0 |

[†]numéro d'enregistrement auprès de la banque de données de *Chemical Abstracts Service.* NA : non applicable.

Un sel de donépézil préféré est le chlorhydrate de donépézil.
Un sel de galantamine préféré est bromhydrate de galantamine.
Un sel de rivastigmine préféré est le tartrate de rivastigmine.
Un sel de FENM préféré est le chlorhydrate de FENM.
Un sel de FENM particulièrement préféré est le bromhydrate de FENM.
Le terme « combinaison », au sens de la présente invention désigne un traitement dans lequel au moins de la FENM et au moins un inhibiteur d'acétylcholinestérase est coadministré à un sujet pour provoquer un effet biologique. Dans une thérapie combinée selon l'invention, ces au moins deux composés peuvent être administrés ensemble ou séparément, en même temps ou séquentiellement. Notamment, la FENM et ledit au moins un inhibiteur d'acétylcholinestérase peuvent être administrés en suivant des voies et/ou des protocoles d'administration différents. En conséquence, bien qu'ils puissent être formulés ensemble, les composés en tant qu'éléments d'une combinaison au sens de l'invention peuvent également être formulés séparément. Par exemple, la FENM peut être administrée oralement et ledit au moins un inhibiteur d'acétylcholinestérase de la combinaison selon l'invention peut être injecté audit sujet, par exemple, par voie intraveineuse, sous cutanée ou encore transdermale. Dans un autre mode de réalisation, par exemple, la FENM peut être administrée oralement et ledit au moins un inhibiteur d'acétylcholinestérase peut être également administré oralement audit sujet concomitamment ou de manière décalée dans le temps. De manière préférée, la séquence d'administration des principes actifs de la combinaison (la FENM et le dit au moins un inhibiteur d'acétylcholinestérase) est telle que lesdits principes actifs ou leur(s) métabolite(s) actif(s) exercent leurs effets biologiques au même moment, de manière que le sujet bénéficie de l'effet maximal de ladite combinaison. Ainsi, de manière particulièrement préférée, la FENM et ledit au moins un inhibiteur d'acétylcholinestérase sont administrés de façon à atteindre leur concentration maximale dans le plasma ou le liquide céphalorachidien, de préférence le liquide céphalorachidien, au même moment.

Le terme de synergie appliqué aux combinaisons selon l'invention, s'entend de combinaisons pour lesquelles les effets pro-mnésiques ou anti-amnésiques observés ou connus pour chacun des principes actifs de ladite combinaison s'additionnent ou se multiplient quand utilisés de manière à ce que leurs effets physiologiques interagissent. Cet effet de synergie permet notamment d'obtenir des effets pro-mnésiques ou anti-amnésiques à des doses auxquelles les principes actifs appliqués dans des monothérapies n'auraient aucun effet ou un effet limité, ce qui peut permettre de diminuer les effets secondaires et/ou d'éviter ou de diminuer la durée des protocoles d'augmentation de doses. En outre, cette synergie entre les effets des composés de la combinaison selon l'invention permet également de potentialiser ces effets pro-mnésiques ou anti-amnésiques ou d'améliorer des types de mémoire complexes comme le montre la partie expérimentale. Les méthodes de l'analyse de l'interaction entre deux molécules et de détermination de l'effet synergique d'une combinaison de composés sont bien connues de l'homme de l'art. Un exemple non limitatif en est l'analyse des isobologrammes et la détermination de l'index de combinaison selon les principes énoncés par Fraser (1872) et tels que mis en œuvre, par exemple, par Martin *et al.* (2020). Ainsi une présence de synergie peut être détectée en appliquant la méthode mathématique illustrée dans la partie expérimentale. La synergie peut être démontrée *in vivo* dans des modèles animaux d'une pathologie, par exemple via des tests d'évaluation des performances cognitives ou des altérations morphologiques associée à la pathologie. La synergie peut être démontrée *in vitro,* par exemple, dans le cadre de tests *in vitro* sur cellules, dans des modèles reconnus par la communauté scientifique tels que par exemple des tests de cytoxicité, en mesurant des paramètres variés relatifs à la viabilité cellulaire, et dans le cas de cellules neuronales, la croissance des neurites, la formation des synapses etc... Concernant la démence et/ou la MA, des illustrations de ces tests sont données, par exemple, par Chumakov *et al.* (2015).

Par « sujet », on entend décrire ici n'importe quel membre du règne animal, de manière préférée les mammifères et de manière encore plus préférée l'homme. Un sujet ayant besoin des traitements combinatoires de l'invention se définit comme un sujet souffrant, étant suspecté de souffrir ou étant considéré à risque de souffrir d'une pathologie neurodégénérative entrainant une démence et des troubles cognitifs associés. Ces pathologies sont, par exemple, les tauopathies, synucléinopathies, les amyloïdopathies, la maladie d'Alzheimer, la maladie de Parkinson, l'atrophie multisystématisée, la maladie de Huntington, l'atrophie corticale postérieure, la maladie de Pick, l'épilepsie, la démence vasculaire, la démence fronto-temporale, la démence à corps de Lewy, la sclérose latérale amyotrophique. Les compositions, combinaisons et méthodes de la présente invention sont particulièrement adaptées au traitement des troubles cognitifs associés à la maladie d'Alzheimer à son stade précoce, modéré ou avancé. Ainsi, dans un mode de réalisation particulier, ledit sujet souffre, est suspecté de souffrir ou est considéré à risque de souffrir de la maladie d'Alzheimer à son stade précoce, modéré ou avancé. Dans un autre mode de réalisation particulier, ledit sujet souffre, est suspecté de souffrir ou est considéré à risque de souffrir de troubles cognitifs associés aux stades précoce, modéré ou avancé de la maladie d'Alzheimer, préférentiellement de troubles cognitifs associés au stade précoce de la maladie d'Alzheimer.

Tel qu'utilisé ici, le terme « traitement » comprend la thérapie, la prévention, la prophylaxie, le retard ou la réduction des symptômes provoqué par ou des causes des maladies ou troubles ci-dessus. Le terme traitement comprend notamment le contrôle de la progression de la maladie et des symptômes associés. Le terme traitement comprend notamment une protection contre les effets de la toxicité causée par l'amyloïde β, ou une réduction ou un retard de ces effets chez les sujets traités. Le terme traitement désigne particulièrement une amélioration, un arrêt ou un retardement de l'évolution des symptômes cognitifs des maladies suscitées.

Combinaison de FENM et d'au moins un inhibiteur de l'acétylcholinestérase

Comme le montrent les données expérimentales présentées ci-dessous, il a été découvert une interaction synergique des effets pro-mnésiques ou anti-amnésique de la FENM et des inhibiteurs d'acétylcholinestérase, qui résulte en une amélioration des symptômes cognitifs dans un modèle animal expérimental d'intoxication au peptide amyloïde

Aβ$_{25-35}$. L'effet protecteur de cette combinaison contre le déclin cognitif engendré par l'intoxication au peptide amyloïde Aβ$_{25-35}$ est observé aussi bien pour la mémoire de travail à court terme que pour la mémoire à long terme.

Ainsi, dans un premier aspect, l'invention se rapporte à une combinaison synergique de la FENM ou l'un quelconque de ses sels pharmaceutiquement acceptable et d'au moins un inhibiteur de l'acétylcholinestérase ou l'un quelconque de ses sels pharmaceutiquement acceptable.

Dans un mode de réalisation particulier, ledit au moins un inhibiteur de l'acétylcholinestérase est sélectionné parmi le donépézil, la galantamine, la rivastigmine ou encore la tacrine. Le donépézil, la galantamine et la rivastigmine sont particulièrement préférés car ils sont autorisés depuis plus de vingt années, leurs effets secondaires et leurs propriétés pharmacologiques sont donc bien connus. La tacrine, du fait de son hépatotoxicité est moins préférée. Néanmoins l'effet synergique mis en évidence ici qui concerne la protection contre les symptômes cognitifs, peut permettre de considérer l'utilisation de doses faibles auxquelles cette hépatotoxicité n'est pas observée. La combinaison synergique de la FENM avec le donépézil est particulièrement préférée.

Comme évoqué, l'effet synergique observé pour les combinaisons de l'invention permet d'envisager d'utiliser, chez l'homme, les doses minimales voire des doses inférieures à ces doses minimales auxquelles les inhibiteurs de l'acétylcholinestérase sont utilisés chez l'homme.

Ainsi dans un mode de réalisation particulier, dans la combinaison synergique selon l'invention, l'au moins un inhibiteur de l'acétylcholinestérase est le donépézil et est administré à une dose inférieure ou égale à 10 mg par jour, inférieure ou égale à 5 mg par jour, inférieure ou égale à 2,5 mg par jour, mais toujours suffisante pour observer un effet bénéfique de la combinaison sur les capacités cognitives du sujet.

Dans un autre mode de réalisation particulier, dans la combinaison synergique selon l'invention, l'au moins un inhibiteur de l'acétylcholinestérase est la galantamine et est administré à une dose inférieure ou égale à 16 mg par jour, inférieure ou égale à 8 mg par jour, inférieure ou égale à 4 mg par jour voire inférieure à 2 mg par jour, mais toujours suffisante pour observer un effet bénéfique de la combinaison sur les capacités cognitives du sujet.

Dans un autre mode de réalisation particulier, dans la combinaison synergique selon l'invention l'au moins un inhibiteur de l'acétylcholinestérase est la rivastigmine et est administré à une dose inférieure ou égale à 3 mg par jour, inférieure ou égale à 1,5 mg par jour, voire inférieure ou égale à 1 mg par jour, mais toujours suffisante pour observer un effet bénéfique de la combinaison sur les capacités cognitives du sujet.

Dans un mode de réalisation particulier, la FENM, dans la combinaison synergique selon l'invention, est utilisée une dose inférieure ou égale à 20 mg par jour, inférieure ou égale à 10 mg par jour, voire inférieure ou égale à 5 mg par jour, mais toujours suffisante pour observer un effet bénéfique de la combinaison sur les capacités cognitives du sujet.

Les capacités cognitives des sujets humains et leur évolution peuvent être mesurée par des tests bien connus de l'homme de l'art. Les tests couramment utilisés pour l'évaluation cognitive des sujets humains, sont par exemple, le Mini-Mental State Examination (MMSE ou test de Folstein), Modified Mini-Mental State Examination ( ou échelle 3MS), Abbreviated Mental Test Score (AMTS ou Test mental abrégé), Dementia questionnaire for persons with Mental Retardation (ou questionnaire DMR), Cognitive Abilities Screening Instrument (CASI ou Instrument de dépistage des capacités cognitives), Trail- making test, Clock drawing test, Alzheimer's disease assessment scale - Cognition (ADAS-Cog ou Sous échelle cognitive de l'échelle de l'évaluation de la maladie d'Alzheimer), General Practitioner Assessment of Cognition (GPCOG ou évaluation par un praticien de la cognition), Montreal Cognitive Assessment (MoCA ou évaluation cognitive de Montréal), ou Rowland Universal Dementia Assessment Scale (RUDAS ou échelle Rowland d'évaluation de la démence universelle), ou encore Alzheimer's Disease Cooperative Study - Activities of Daily Living (ADCS-ADL ou étude coopérative de la maladie d'Alzheimer-activité de la vie quotidienne) selon leur dénomination en langue anglaise.

Plus particulièrement, le MMSE permet de dépister les personnes souffrant d'une atteinte neurocognitive majeure (démence) sans la rattacher à une pathologie particulière. Le MMSE sert également à assurer les suivis de l'état cognitif des personnes et à mesurer le déclin des fonctions cognitives chez les personnes qui souffrent d'atteintes neurocognitives. Ce test évalue l'orientation, l'enregistrement, l'attention et le calcul, la rétention mnésique, le langage et praxie de construction. Le CERAD (dans sa dénomination en langue anglaise Consortium to Establish a Registry for Alzheimer's Disease ou Consortium pour établir un registre de la maladie d'Alzheimer) a établi une échelle de sévérité de la démence associée aux scores obtenus dans le MMSE. Un score entre 19 et 24 est associé à une démence légère, entre 10 et 18, une démence modérée, et un score inférieur à 10 correspond à une démence sévère, le score maximal étant de 30. Une variation de 2 points du score est généralement considérée comme cliniquement relevante.

L'ADAS-Cog est une sous-échelle cognitive de l'échelle d'évaluation de la maladie d'Alzheimer et ne concerne donc que les aspects de la démence liés à la cognition. Ainsi il peut être utilisé pour évaluer (*i.e.* scorer) et suivre l'évolution de tout type de démence. L'ADAS-Cog évalue l'orientation, la mémoire, la fonction exécutive, la capacité visuospatiale, le langage ou la pratique, avec une plage de scores de 0 à 70, un score plus élevé indiquant une plus grande déficience. L'ADAS-Cog est considéré comme plus sensible que le MMSE. C'est un des tests les plus communément utilisés pour l'évaluation clinique des composés candidats à l'obtention d'une Autorisation de Mise sur le Marché dans le cadre de traitements anti-démence et également dans la mesure de l'évolution des atteintes cognitives.

Ainsi une combinaison avec un effet bénéfique de la combinaison sur les capacités cognitives du sujet montrera un ralentissement, ou une stabilisation de la détérioration des capacités cognitives du sujet au regard de l'évolution usuelle établie chez des sujets non traités au même stade de la maladie pour une période de temps donnée. En d'autres termes, par exemple, dans le cas de l'ADAS-Cog, un effet bénéfique sur les capacités cognitives du sujet correspondra à une diminution, une stabilisation ou une augmentation de plus faible intensité de du score de l'ADAS-Cog comparée à l'aggravation usuelle du score observée chez des sujets non traités à un stade équivalent et d'un âge équivalent. Bien que l'aggravation du score dépende du stade de la pathologie et de l'âge du sujet, une augmentation annuelle de 5,82 points du score de l'ADAS-Cog est globalement observée chez les sujets souffrant de la maladie d'Alzheimer non traités (Zhang *et al.,* 2020). Concernant le MMSE, par exemple, un effet bénéfique sur les capacités cognitives du sujet correspondra à une augmentation, une stabilisation ou une diminution de plus faible intensité du score MMSE comparée à l'aggravation usuelle du score MMSE observée chez des sujets non traités. Une diminution annuelle de 2,28 points du score du MMSE est globalement observée chez les sujets souffrant de la maladie d'Alzheimer non traités (Rossetti *et al.* 2010).

De manière surprenante, la synergie entre la FENM et l'au moins un inhibiteur de l'acétylcholinestérase est observée pour un ratio molaire FENM/inhibiteur de l'acétylcholinestérase très faible. En effet, le ratio molaire testé pour la mémantine et le donépézil dans l'état de l'art est supérieur à 4, si l'on considère les combinaisons à base de 20 mg de mémantine et de 10 mg de donépézil) c'est notamment le ratio contenu dans NAMZARIC® autorisé par la FDA, à base de chlorhydrate de donépézil et de chlorhydrate de mémantine. A noter que les valeurs de Tmax chez l'homme concernant le donépézil et la mémantine quand administrés oralement sont comparables : entre 3 et 8 heures pour la mémantine (Maekawa et al., 2019) et de $4,1 \pm 1,5$ heures pour le donépézil (Rogers & Friedoff, 1998). Les données expérimentales montrent que l'effet synergique découvert pour la combinaison de l'invention est obtenu à un ratio molaire bien inférieur, pouvant même être inversé, signant la spécificité des combinaisons synergiques à base de FENM.

Ainsi dans un mode de réalisation particulier de la combinaison synergique de la FENM et d'au moins un inhibiteur de l'acétylcholinestérase selon l'invention, le ratio molaire FENM/inhibiteur de l'acétylcholinestérase est inférieur ou égal à 4, inférieur ou égal à 3, inférieur ou égal à 2, de préférence inférieur ou égal à 1, inférieur ou égal à 0,8, voire inférieur ou égal à 0,5. Ledit ratio molaire FENM/inhibiteur de l'acétylcholinestérase étant supérieur ou égal à 0,1. De manière particulièrement préférée, ledit ratio molaire FENM/inhibiteur de l'acétylcholinestérase est inférieur à 1 et supérieur ou égal à 0,1. Dans un autre mode de réalisation particulier ledit inhibiteur de l'acétylcholinestérase est le donépézil et le ratio molaire FENM/donépézil est inférieur ou égal à 4, est inférieur ou égal à 3, inférieur ou égal à 2, de préférence inférieur ou égal à 1, inférieur ou égal à 0,8, voire inférieur ou égal à 0,5. Ledit ratio molaire FENM/donépézil étant supérieur ou égal à 0,1. De manière particulièrement préférée, ledit ratio molaire FENM/ donépézil est inférieur à 1 supérieur ou égal à 0,1.

<u>Composition selon l'invention comprenant une combinaison synergique de FENM et d'au moins un inhibiteur de l'acétylcholinestérase.</u>

Dans un autre aspect, l'invention se rapporte à une composition comprenant une combinaison synergique telle que décrite ci-dessus.

Dans cette composition, la FENM ou l'un quelconque de ses sels pharmaceutiquement acceptable et l'au moins un inhibiteur de l'acétylcholinestérase ou l'un quelconque de ses sels pharmaceutiquement acceptable sont formulés ensemble ou séparément.

Dans un mode particulier la FENM et l'au moins l'au moins un inhibiteur de l'acétylcholinestérase sont les seuls principes actifs de ladite composition. En d'autres termes, la FENM et l'au moins un inhibiteur de l'acétylcholinestérase sont les seuls composés au sein de la composition ayant une activité thérapeutique ou de prévention.

De préférence, la composition est administrée au sujet sous la forme de préparation pharmaceutique, par exemple, sans être limitatif, de manière orale, locale (cutanée, buccale, sublinguale) ou de manière parentérale (sous-cutanée, intramusculaire ou par intraveineuse). L'administration orale est particulièrement préférée.

Les quantités des ingrédients actifs au sein de cette composition, c'est à dire au moins la FENM et l'au moins un inhibiteur de l'acétylcholinestérase, sont compatibles avec les doses telles que déterminées ci-dessus pour les combinaisons synergiques de l'invention. En d'autres termes, quand ladite composition se présente sous forme de dosage unitaire (sous forme de cachet, capsule, poudre, émulsion, solution) ledit dosage comprend des quantités de FENM ou de l'au moins un inhibiteur de l'acétylcholinestérase qui sont un multiple ou un diviseur des doses déterminées pour les combinaisons synergiques de l'invention, permettant ainsi d'obtenir en une ou plusieurs prises le dosage adéquat tel que défini ci-dessus. Dans un mode de réalisation particulier, la composition selon l'invention comprend 10 mg de donépézil, 7,5 mg de donépézil, 5 mg de donépézil, voire 2,5 mg de donépézil. Dans un autre mode de réalisation particulier, la composition selon l'invention comprend 16 mg de galantamine, 8 mg de galantamine, 4 mg de galantamine, voire 2 mg de galantamine. Dans un autre mode de réalisation particulier, la composition selon l'invention comprend 3 mg de rivastigmine, 1,5 mg de rivastigmine, voire 1 mg de rivastigmine.

Dans un mode de réalisation particulier, la composition selon l'invention comprend 20 mg de FENM, 10 mg de FENM, 7,5 mg de FENM, 5 mg de FENM, voire 2,5 mg de FENM.

Dans un autre mode de réalisation, la composition selon l'invention comprend 20 mg de FENM et 10 mg de donépézil, 7,5 mg de donépézil, 5 mg de donépézil, voire 2,5 mg de donépézil. Dans un autre mode de réalisation, la composition selon l'invention comprend 10 mg de FENM et 10 mg de donépézil, 7,5 mg de donépézil, 5 mg de donépézil, voire 2,5 mg de donépézil. Dans un autre mode de réalisation, la composition selon l'invention comprend 7,5 mg de FENM et 10 mg de donépézil, 7,5 mg de donépézil, 5 mg de donépézil, voire 2,5 mg de donépézil. Dans un autre mode de réalisation, la composition selon l'invention comprend 5 mg de FENM et 10 mg de donépézil, 7,5 mg de donépézil, 5 mg de donépézil, voire 2,5 mg de donépézil. Dans un autre mode de réalisation, la composition selon l'invention comprend 2,5 mg de FENM et 10 mg de donépézil, 7,5 mg de donépézil, 5 mg de donépézil, voire 2,5 mg de donépézil.

Dans un autre mode de réalisation, dans la composition selon l'invention, le ratio molaire FENM/inhibiteur de l'acétylcholinestérase est inférieur ou égal à 4, inférieur ou égal à 3, inférieur ou égal à 2, de préférence inférieur ou égal à 1, inférieur ou égal à 0,8, voire inférieur ou égal à 0,5. Ledit ratio est supérieur ou égal à 0,1. De manière particulièrement préférée, ledit ratio molaire FENM/inhibiteur de l'acétylcholinestérase est inférieur à 1 et supérieur ou égal à 0,1. Dans un autre mode de réalisation particulier, ledit inhibiteur de l'acétylcholinestérase est le donépézil et le ratio molaire FENM/donépézil est inférieur ou égal à 4, est inférieur ou égal à 3 est inférieur ou égal à 2, de préférence inférieur ou égal à 1, inférieur ou égal à 0,8, voire inférieur ou égal à 0,5, ledit ratio étant supérieur ou égal à 0,1. De manière particulièrement préférée, ledit ratio molaire FENM/ donépézil est inférieur à 1 et supérieur ou égal à 0,1.

La dose peut être administrée en plusieurs prises réparties dans la journée, le nombre de prises dans la journée permettant d'obtenir la dose journalière désirée. Ainsi dans un mode de réalisation particulier, les doses en question peuvent être administrées en une à quatre prises journalières, par exemple 1 fois, par exemple 2 fois, par exemple 3 fois, voire 4 fois.

Dans un mode préféré de réalisation, dans ladite composition, la combinaison de la FENM ou de l'au moins un inhibiteur de l'acétylcholinestérase est conditionnée de manière à fournir la dose correspondant à une prise sans nécessiter de manipulation telle qu'une mesure de volume, une pesée ou la division d'une tablette, ce qui est particulièrement avantageux chez des sujets présentant des atteintes cognitives puisque cela évite tout calcul ou manipulation particulière.

Par exemple, FENM ou de l'au moins un inhibiteur de l'acétylcholinestérase sont formulés séparément sous forme de poudre, micro-granules, granules voire de cachets distincts puis associés dans une gélule pour en faciliter la prise.

Les compositions pharmaceutiques peuvent être formulées selon la pratique pharmaceutique conventionnelle (voir, par exemple, Remington: The Science and Practice of Pharmacy (23rd ed.), ed. A Adeboye Adejare, 2020) et les caractéristiques PK/PD des principes actifs. Classiquement, dans les compositions selon l'invention la FENM et/ou ledit au moins un inhibiteur d'acétylcholinestérase est mélangé avec un excipient pharmaceutiquement acceptable.

Dans un mode de réalisation, ladite composition se présente sous forme de tablette ou comprimé. Ladite tablette ou comprimé peut être sécable en 1, 2, 3, voire 4 morceaux de manière à pouvoir fournir au sujet la dose nécessaire à une prise en utilisant 1, 2 ou 3 morceaux de ladite tablette. Cela est particulièrement intéressant, par exemple, dans le cas où le traitement nécessite une période d'augmentation de dose, pour arriver à la dose journalière ciblée, les morceaux pouvant correspondre aux paliers d'augmentation, et la tablette ou comprimé entier à la dose cible du traitement.

Dans un mode de réalisation, dans ladite composition, la FENM et l'au moins un inhibiteur de l'acétylcholinestérase sont mélangés au sein de la même tablette ou comprimé avec le(s) même(s) excipients. Dans un mode de réalisation particulier, la FENM et l'au moins un inhibiteur de l'acétylcholinestérase sont présents dans des compartiments différents de ladite tablette ou comprimé avec des excipients qui leur sont spécifiques, et qui sont fonction de leurs propriétés physicochimiques ou de leurs propriétés pharmacocinétiques.

Dans un autre mode de réalisation, particulièrement intéressant en cas de molécules aux profils PK/PD différents, la FENM et l'au moins un inhibiteur de l'acétylcholinestérase sont présents séparés au sein de cette tablette. Ainsi, dans un mode de réalisation particulier, la FENM et l'au moins un inhibiteur de l'acétylcholinestérase sont présents dans des compartiments différents de ladite tablette ou comprimé, par exemple un composé étant situé à l'extérieur et l'autre composé à l'intérieur de ladite tablette ou comprimé, ce qui permet une administration séparée et décalée dans le temps de ces composés.

Dans un mode de réalisation de la composition selon l'invention, la FENM et/ou l'au moins un inhibiteur de l'acétylcholinestérase peuvent être formulés pour être libéré(s) sensiblement immédiatement après administration, à tout moment, ou à une période de temps prédéterminé après administration c'est-à-dire formulé(s) de manière à être libérés de manière contrôlée dans l'organisme. Les formulations à libération contrôlée comprennent (i) des formulations qui créent une concentration sensiblement constante du composé ou de son dérivé actif dans le corps sur une période de temps prolongée; (ii) des formulations qui, après un temps de latence prédéterminé, créent une concentration sensiblement constante du composé ou de son dérivé actif dans le corps sur une période de temps prolongée ; (iii) des formulations qui maintiennent l'action du composé ou de son dérivé actif pendant une période de temps prédéterminée en maintenant un niveau dudit composé ou de son dérivé actif relativement, constant et efficace dans le corps permettant en outre une minimisation concomitante des effets secondaires indésirables associés aux fluctuations du taux plasmatique dudit composé ou de son dérivé actif; (iv) des formulations qui localisent l'action du composé ou de son dérivé actif, par exemple, à proximité ou dans le tissu ou l'organe malade, ou dans un compartiment corporel spécifique; et (v) des formulations qui ciblent l'action du dudit composé ou de son dérivé actif en utilisant des supports ou des dérivés chimiques pour délivrer le médicament à un type de cellule cible particulier.

L'administration de composés sous la forme d'une formulation à libération contrôlée est particulièrement préférée dans les cas où le composé a (i) un index thérapeutique étroit (c'est-à-dire la différence entre la concentration plasmatique conduisant à des effets nocifs, des effets secondaires ou des réactions toxiques, et la concentration plasmatique conduisant à un effet thérapeutique est faible; en général, l'index thérapeutique, TI, est défini comme le rapport de la dose létale médiane (DL50) à la dose efficace médiane (DE50)); (ii) une étroite fenêtre d'absorption dans le tractus gastro-intestinal; ou (iii) une demi-vie biologique très courte de sorte que des administrations fréquentes sont nécessaires pour maintenir le niveau plasmatique à un niveau thérapeutique efficace. Différentes stratégies peuvent être poursuivies afin d'obtenir une libération avec une vitesse de libération du composé ou de son dérivé actif adaptée au métabolisme du médicament en question. La libération contrôlée peut être obtenue par une sélection appropriée de divers paramètres et ingrédients de formulation, y compris, par exemple, divers types de compositions à libération contrôlée et revêtements connues de l'homme de l'art. Ainsi, le composé est formulé avec des excipients appropriés en une composition pharmaceutique qui, lors de l'administration, libère ledit composé de manière contrôlée (compositions unitaires ou multiples de comprimés ou de gélules, solutions huileuses, suspensions, émulsions, microcapsules, microsphères, nanoparticules, patchs et liposomes).

Dans un mode de réalisation encore plus particulier des moyens techniques spécifiques tels que des réservoirs, pompes ou patchs transdermiques (en d'autres termes un timbre autocollant qui dispense une substance par voie percutanée) peuvent contribuer à la libération contrôlée de au moins la FENM et/ou de l'au moins un inhibiteur de l'acétylcholinestérase.

Dans un autre mode de réalisation, ladite composition est formulée sous forme liquide. Elle peut être conditionnée sous forme de dose unitaire dans des contenants tels que des ampoules, ou alors dans un contenant tel qu'une bouteille ou un flacon associé à un dispositif permettant le prélèvement et, optionnellement, l'administration du volume désiré pour obtenir la dose adéquate.

Utilisations thérapeutiques de la combinaison synergique ou de la composition selon l'invention.

L'effet synergique nouveau découvert par les inventeurs pour la combinaison de la FENM avec un moins inhibiteur de l'acétylcholinestérase telle que décrit dans la partie expérimentale constitue une nouvelle solution thérapeutique.

Ainsi selon un autre aspect, l'invention se rapporte particulièrement à la composition ou à la combinaison synergique telles que décrites précédemment dans tous leurs modes de réalisation, pour leur utilisation en tant que médicament. Dans un mode de réalisation préféré, l'invention se rapporte la combinaison synergique de FENM et de donépézil ou une composition la comprenant, telles que décrites ci-dessus dans tous leurs modes de réalisation, pour leur utilisation en tant que médicament.

Selon un aspect supplémentaire, l'invention se rapporte particulièrement à la composition ou à la combinaison synergique, telles que décrites précédemment, pour leur utilisation dans le traitement d'une pathologie sélectionnée parmi les tauopathies, synucléinopathies, les amyloïdopathies, la maladie d'Alzheimer, la maladie de Parkinson, l'atrophie multisystématisée, la maladie de Huntington, l'atrophie corticale postérieure, la maladie de Pick, l'épilepsie, la démence vasculaire, la démence fronto-temporale, la démence à corps de Lewy, la sclérose latérale amyotrophique. Dans un mode de réalisation particulier, ledit traitement se rapporte au traitement de la maladie d'Alzheimer à son stade précoce, modéré ou avancé, de préférence la maladie d'Alzheimer à son stade précoce. Dans un mode de réalisation particulier, ledit traitement se rapporte au traitement des troubles cognitifs associés à l'une quelconque des pathologies sélectionnées parmi une tauopathie, une synucléinopathie, une amyloïdopathie, la maladie d'Alzheimer, la maladie de Parkinson, l'atrophie multisystématisée, la maladie de Huntington, l'atrophie corticale postérieure, la maladie de Pick, l'épilepsie, la démence vasculaire, la démence fronto-temporale, la démence à corps de Lewy, la sclérose latérale amyotrophique.

Un mode de réalisation particulier concerne la composition ou la combinaison synergique telles que décrites précédemment pour leur utilisation dans le traitement des troubles cognitifs associés au stade précoce, modéré ou avancé de la maladie d'Alzheimer, de préférence au stade précoce. En effet les données expérimentales montrent un effet synergique particulièrement important dans le traitement des atteintes de la mémoire contextuelle à long terme, ce qui présente un avantage particulier dans le cas des patients au stade précoce de la maladie.

Un autre mode de réalisation particulier concerne la composition ou la combinaison synergique telles que décrites précédemment pour leur utilisation des altérations :

- de la mémoire à court terme,

- de la mémoire à moyen terme,
- de la mémoire spatiale, ou
- des capacités de reconnaissance et/ou d'apprentissage,

associées à l'une quelconque des pathologies sélectionnées parmi une tauopathie, une synucléinopathie, une amyloïdopathie, la maladie d'Alzheimer, la maladie de Parkinson, l'atrophie multisystématisée, la maladie de Huntington, l'atrophie corticale postérieure, la maladie de Pick, l'épilepsie, la démence vasculaire, la démence fronto-temporale, la démence à corps de Lewy, la sclérose latérale amyotrophique. Dans un mode particulier lesdites altérations de la mémoire à court terme, de la mémoire à moyen terme, de la mémoire spatiale, ou des capacités de reconnaissance et/ou d'apprentissage sont associées au stade précoce, modéré ou avancé de la maladie d'Alzheimer, de préférence au stade précoce.

Selon un autre aspect, l'invention se rapporte à une méthode de traitement d'une pathologie sélectionnée parmi les tauopathies, synucléinopathies, les amyloïdopathies, la maladie d'Alzheimer, la maladie de Parkinson, l'atrophie multisystématisée, la maladie de Huntington, l'atrophie corticale postérieure, la maladie de Pick, l'épilepsie, la démence vasculaire, la démence fronto-temporale, la démence à corps de Lewy, la sclérose latérale amyotrophique comprenant l'administration à un sujet en ayant besoin, d'une combinaison synergique de la FENM ou l'un quelconque de ses sels pharmaceutiquement acceptables avec au moins un inhibiteur de l'acétylcholinestérase ou l'un quelconque de ses sels pharmaceutiquement acceptables.

Dans ladite méthode, ladite combinaison synergique est telle que décrite précédemment, dans tous ses modes de réalisation. Dans un mode de réalisation, ladite méthode peut comprendre l'administration d'une composition selon l'invention telle que décrite ci-dessus.

Dans un mode particulier, ladite méthode comprend l'administration séparée, concomitante ou séquentielle, de la FENM et de l'au moins un au moins un inhibiteur de l'acétylcholinestérase. Dans ce mode de réalisation, la FENM et l'au moins un au moins un inhibiteur de l'acétylcholinestérase peuvent être administrés séparément au sujet par des voies identiques, telles que par exemple par la voie orale, parentérale ou transdermique. Dans un mode alternatif de réalisation, la FENM et l'au moins un au moins un inhibiteur de l'acétylcholinestérase peuvent être administrés au sujet par des voies différentes. Par exemple l'un par voie orale et l'autre par la voie transdermique ou parentérale. Un exemple particulier étant constitué par la forme transdermique de la rivastigmine commercialisée sous le nom Exelon® ou du donépézil (en essais clinique aux États-Unis sous le nom de Adlarity®).

**Exemples**

Abréviations

A$\beta_{25-35}$ : fragment de 11 acides aminés de séquence Nt-GSNKGAIIGLM-Ct (SEQ ID NO 1) du peptide APP.

FENM : Bromhydrate de 3(2-Fluoroéthyl)tricyclo [3.3.1.13,7]decan-1-amine (aussi bromhydrate de fluoroéthylnormémantine).

DPZ : chlorhydrate de donépézil.

YMT : test d'alternance spontanée dans le labyrinthe en Y ; « Y-maze test » en langue anglaise.

PAT : test d'évitement passif ; « Passive Avoidance Test » ou « step-through passive avoidance test » en langue anglaise.

ICV : intracérébroventriculaire.

IP : intrapéritonéal(e).

IC : indice de combinaison.

iChE : inhibiteur d'acétylcholinestérase.

PP : pourcentage de protection.

SNC : Système Nerveux Central.

MA : Maladie d'Alzheimer.

## 1. Matériel et méthodes

Les expérimentations animales sont conduites en accord avec les dispositions de la directive de l'Union Européenne N° 2010/63 et ont été dûment autorisées par le Comité Consultatif National d'Ethique (CCNE) de la République française et les directives ARRIVE (Kilkenny *et al.,* 2010).

### 1.1. Animaux

Les expérimentations *in vivo* ont été menées sur des souris mâles Swiss OF-1 (Janvier, St Berthevin, France) âgées de 7 à 9 semaines d'une masse de 32± 2 g. Les animaux sont hébergés par groupe de 8 à 10 individus dans des cages en plastiques avec un accès à volonté à la boisson et à la nourriture, dans un environnement contrôlé (cycle jour/nuit de 12 h, la lumière s'allumant à 7h00 et les expérimentations comportementales se déroulant entre 9h00 et 17h00), sous atmosphère et environnement sonore contrôlés.

### 1.2. Composés tests et peptides

**Solutions stocks de DPZ et FENM.**

Le DPZ a été obtenu auprès de Eisai Co. Ltd (Tokyo, Japon). La FENM a été obtenue auprès de M2i Life Sciences (Saint-Cloud, France). Les solutions stock des composés ont été obtenues par solubilisation en tampon NaCl (0,9%, véhicule) à une concentration de 2 mg/mL qui correspond à une dose de 5 mg/Kg. Ces solutions stocks sont conservées à 4°C pendant 2 semaines maximum.

**Solution stock de peptide amyloïde [25-35] ; formation des oligomères.**

Le peptide amyloïde [25-35], noté $A\beta_{25-35}$ (Eurogentec, Angers, France), a été solubilisé dans de l'eau distillée stérile à une concentration de 3 mg/mL, la solution stock ainsi formée est aliquotée et conservée à -20°C jusqu'à utilisation.
Les oligomères de $A\beta_{25-35}$ sont formés tel que décrit par Maurice *et al.* (1996), lors d'une incubation à 37°C pendant 4 jours avant injection aux animaux. La solution véhicule ou le peptide contrôle sont soumis au même traitement avant administration. Il a déjà été démontré que l'injection de solution véhicule (eau distillée) donne la même absence d'effet que l'injection de peptide contrôle Sc Aβ (peptide contrôle), qui comprend les mêmes acides aminés que $A\beta_{25-35}$ dans un ordre aléatoire et qui ne s'oligomérise pas.

**Administration aux animaux**

Les composés sont administrés IP dans un volume de 100 µL par 20g de masse corporelle. L'administration de la combinaison FENM/DPZ s'effectue dans un volume de 100 µL par 20g de masse corporelle. Les doses de composés mentionnés dans la partie expérimentale correspondent aux doses des sels des composés utilisés et non à l'équivalent massique du composé en soi. Les doses administrées exprimées en moles sont mentionnées dans le tableau 2. Pour chaque composé les doses administrées sont 0,01 ; 0,03 ; 0,1 ; 0,3 et 1 mg/Kg. Dans le cas de la combinaison, les doses de donépézil et de FENM coadministrées étaient respectivement de 0,03 et 0,01 mg/Kg.
Les doses correspondantes exprimées en mMol/Kg sont reportées au tableau 2 ci-dessous.

Tableau 2

| Principe actif | Dose en mg/Kg dans les expérimentations | Dose en mMol/Kg |
|---|---|---|
| | 0,01 | 0,04 |
| | 0,03 | 0,11 |
| fluoroéthylnormémantine | 0,1 | 0,36 |
| | 0,3 | 1,08 |
| | 1 | 3,60 |

(suite)

| Principe actif | Dose en mg/Kg dans les expérimentations | Dose en mMol/Kg |
|---|---|---|
| donépézil | 0,03 | 0,07 |
| | 0,1 | 0,24 |
| | 0,3 | 0,72 |
| | 1 | 2,40 |
| | 3 | 7,21 |

La solution d'oligomères de $A\beta_{25-35}$ et le véhicule (eau distillée stérile) sont administrés aux souris par injection ICV, tel que décrit dans Maurice *et al.* (1996).

**1.3. Tests cognitifs / comportementaux**

Le modèle d'injection ICV d'oligomères d'$A\beta_{25-35}$ est un modèle bien connu dans l'état de l'art comme. Ce modèle est considéré comme un modèle pertinent de criblage pour l'activité neuroprotectrice de composés, et plus particulièrement un modèle de première intention pertinent de la maladie d'Alzheimer. Les oligomères d'$A\beta_{25-35}$ sont connus pour être cytotoxiques pour les cellules neuronales chez la souris et induire des troubles de la mémoire spatiale et de travail. Ce déficit s'accompagne par la génération d'un stress mitochondrial, d'un stress oxydant et d'une apoptose des cellules, notamment dans l'hippocampe, et par une inflammation du système nerveux central.

La capacité des composés et de leur combinaison à diminuer les symptômes cognitifs de la neurodégénération induite par les oligomères $A\beta_{25-35}$, est testée. Pour cela l'administration des composés test a lieu le même jour que l'injection des oligomères $A\beta_{25-35}$ et se poursuit sur une base journalière jusqu'au jour 7 après l'injection des oligomères $A\beta_{25-35}$ ; les souris sont alors soumises aux tests YMT, ou PAT au jour 8 après l'injection des oligomères $A\beta_{25-35}$.

**Test d'évitement passif (PAT)**

Ce test mesure la mémoire non-spatiale (contextuelle) à long-terme. L'appareil utilisé pour ce test est une boîte à deux compartiments (15 x 20 x 15 cm de haut) l'un éclairé avec des parois en PVC blanc et l'autre à l'obscurité avec des parois de PVC noir et un sol grillagé. Une porte guillotine sépare les compartiments. Une lampe de 60 W est positionnée à 40 cm au-dessus de la boite et éclaire le compartiment blanc. Des chocs électriques (0,3 mA pendant 3 s) peuvent être appliqués au sol de la grille *via* un générateur (Lafayette Instruments, Lafayette, USA). Le test comprend une session d'apprentissage et une session de test. La porte à guillotine est fermée pendant la session de formation. Chaque souris est placée dans le compartiment blanc. Après 5 secondes, la porte se lève. Quand la souris entre dans le compartiment obscur de façon que ses quatre pattes soient en contact avec le grillage, la porte se ferme et des décharges électriques sont appliquées pendant 3 secondes. Le temps mis par la souris pour rentrer dans le compartiment obscur (STL-Tg) et le niveau de sensibilité au choc (0= aucun signe ; 1 = sursauts ; sursaut et vocalises) sont relevés. La session de test est effectuée 24 heures après la session d'apprentissage. Chaque souris est replacée dans le compartiment blanc éclairé. Après 5 secondes, la porte se soulève, le temps de latence (STL-R), c'est-à-dire le temps que met la souris pour aller dans le compartiment sombre est mesuré. La durée maximum est 300 secondes. Les souris présentant un STL-Tg et un STL-R < 10 s et un faible niveau de sensibilité ne sont pas prises en compte pour le test. Le taux d'attrition est habituellement de 5%. Une souris ayant des capacités de mémoire altérées aura un STL-R inférieur à celui d'une souris aux capacités normales. Les résultats sont présentés par leur valeur médiane avec les interquartiles (25%-75%).

**Test d'alternance spontanée dans le labyrinthe en Y (YMT)**

Le test d'alternance spontanée est utilisé pour étudier la mémoire spatiale de travail (à très court terme) chez les rongeurs. Le labyrinthe est fait de chlorure de polyvinyle (PVC) opaque gris. Chacun de ses bras mesure 40 cm de long, 13 cm de haut et 3 cm de large à sa base, 10 cm à son sommet. Les bras convergent l'un vers l'autre, avec un angle égal entre les différents bras. Brièvement, chaque souris est placée à l'extrémité d'un bras et est laissée libre de se mouvoir pour une session de 8 min. Les entrées de la souris dans chacun des bras, y compris le bras à l'extrémité duquel elle a été déposée, sont enregistrées. Une alternance est définie comme l'entrée successive de l'animal dans trois bras différents. Le nombre d'alternances maximal est donc le nombre total d'entrées dans chacun des bras moins 2 et le pourcentage d'alternance est calculé selon la formule :

$$\%Alt = \frac{\text{nombre d'alternance réalisées}}{\text{nombre d'alternance maximum}} \times 100$$

Les données des animaux montrant des comportements extrêmes (pourcentage d'alternance < 20% ou > 90%) ne sont pas prises en compte dans les calculs. Le taux d'attrition est d'ordinaire de 5%. En conditions normales, une souris va spontanément alterner les explorations des chacun des bras. Une souris ayant des capacités de mémoire et/ou d'orientation altérées verra son pourcentage d'alternance diminuer.

### 1.4. Analyses statistiques - calcul de l'indice de combinaison (IC)

**Analyses statistiques**

Les analyses ont été effectuées en utilisant Prism v5.0 (GraphPad Software, San Diego, CA, USA). Les données ont été analysées à l'aide d'analyses de variance unidirectionnelles (ANOVA, valeur F), suivies d'un test de Dunnett ou bien une ANOVA de Kruskal-Wallis non-paramétrique (H value) suivie par un test de comparaison de Dunn. Les niveaux statistiques de significativité sont $p < 0.05$, $p < 0.01$ and $p < 0.001$.

**Indice de combinaison**

La nature de l'interaction de deux composés à un niveau d'effet donné a été évaluée par l'analyse des isobologrammes (Martin *et al.,* 2020 ; Maurice, 2016). La représentation des isobologrammes suit le concept de Fraser (1872). Les courbes dose-réponse suivent un effet biphasique, seule la partie ascendante de la courbe a été prise en compte dans les calculs. La concentration requise pour produire un effet donné (par exemple, où ICx = IC50) est déterminée pour le composé A (ICx,A) et le composé B (ICx,B) et indiquée sur les axes x et y d'un tracé à deux coordonnées, formant les deux points, (ICx,A, 0) et (0, ICx,B). La ligne reliant ces deux points est la ligne d'additivité. Dans un mélange de médicaments A + B, les concentrations de A et B contenues dans la combinaison qui produisent le même effet sont représentées par les coordonnées (CA,x, CB,x).

L'indice de combinaison (IC) est calculé comme suit :

$$IC = CA, x/ICx, A + CB, x/ICx, B$$

où CA,x et CB,x sont les concentrations des composés A et B utilisés dans une combinaison qui génère x% de l'effet de combinaison maximal.

IC est l'indice combiné. ICx,A et ICx,B sont les concentrations des composés A et B nécessaires seuls pour produire x% de l'effet maximal. Un IC inférieur/égal à/supérieur à 1 indique respectivement une synergie/une additivité/un antagonisme. Pour calculer l'IC basé sur la représentation des isobologrammes, les pourcentages d'alternance et les latences d'évitement passif ont été exprimés en pourcentage de protection (PP) pour chaque groupe de traitement avec PP(V/V) défini sur 100 % et PP(A$\beta_{25\text{-}35}$/V) sur 0 % (Martin *et al.,* 2020 ; Maurice, 2016).

### 2. Résultats

### 2.1. Effet synergique d'une combinaison FENM-iChE sur la mémoire de travail à court terme (YMT), dans un modèle d'intoxication à A$\beta$.

Les données confirment les effets anti-amnésiques de la FENM et du DPZ sur les atteintes induites par les oligomères A$\beta_{25\text{-}35}$ sur la mémoire de travail à court terme.

Il a d'abord été recherché les doses subefficaces, c'est-à-dire des doses de composés auxquelles, quand ceux-ci sont administrés seuls, un effet sur les atteintes induites par la FNEM à la limite de la significativité est observé ou bien des doses immédiatement inférieures à la première dose significativement active.

Les performances des souris intoxiquées à A$\beta_{25\text{-}35}$ et administrées avec la FENM seule sont significativement améliorées par rapport au témoin dès 0,03 mg/Kg (figure 1). L'amélioration observée pour les souris administrées avec une dose de 0,01 mg/Kg, est à la limite de la significativité statistique. Dès 0,03 mg/Kg la FENM améliore significativement le pourcentage d'alternance des souris intoxiquées avec A$\beta_{25\text{-}35}$ ; en outre ces performances ne sont pas significativement différentes de celles des souris non intoxiquées. L'administration du donépézil seul permet également une amélioration des capacités mnésiques des animaux mesurées par ce test. Aucune amélioration n'est cependant observée à 0,01 mg/Kg (non présenté). Administré à 0,03 mg/Kg le DPZ ne confère pas d'amélioration statistiquement significative des performances des animaux intoxiqués. Administré à 0,1 mg/Kg le DPZ confère une amélioration des symptômes mnésiques, puisque les performances des animaux intoxiqués et traités ne sont pas significativement différentes de celles des animaux non intoxiqués et non traités ; cependant ces performances ne sont pas non plus significativement différentes de celles observées chez les souris intoxiquées non traitées.

Le traitement combiné des souris intoxiquées avec de la FENM et du DPZ à 0,01 et 0,03 mg/Kg respectivement résulte en une amélioration du pourcentage d'alternance qui n'est pas significativement différent de celui des souris non intoxiquées. Le ratio molaire FENM/DPZ dans ce traitement combiné est de 0,5.

Les données relatives à l'index de combinaison pour ces expérimentations sont reportées au tableau 3 ci-dessous.

Tableau 3

| Traitement (mg/kg IP) | PP (%) | $C_{x,FENM}$ | $C_{x,DPZ}$ | CI |
|---|---|---|---|---|
| FENM (0) | $0,0 \pm 18,2$ | | | |
| FENM (0,01) | $38,5 \pm 31,9$ | | | |
| FENM (0,03) | $58,4 \pm 20,8$ | | | |
| FENM (0,1) | $71,2 \pm 33,3^{\dagger}$ | | | |
| DPZ (0) | $0,0 \pm 18,2$ | | | |
| DPZ (0,03) | $10,6 \pm 34,0$ | | | |
| DPZ (0,1) | $46,9 \pm 26,4$ | | | |
| DPZ (0,3) | $58,9 \pm 33,7°$ | | | |
| FENM (0,01) + DPZ (0,03) | $50,7 \pm 30,2$ | $0,063 \pm 0,016$ | $0,236 \pm 0,066$ | **$0,30 \pm 0,08$** |

PP : pourcentage de protection ; CI: indice de combinaison. $C_{x,FENM}$ et $C_{x,DPZ}$ ont été calculés à partir de la droite de régression des courbes dose-réponse de chacun des composés seuls : $^{\dagger}y = 841,34x$, $R^2 = 0,7817$; $°y = 224,69x$, $R^2 = 0,8819$.

Un CI de 0.3 (tableau 3) est mesuré pour la combinaison FENM-DPZ aux concentrations subefficaces. Ce CI marque une forte interaction synergique entre la FENM et le DPZ, comme cela est illustré par l'échelle de pourcentage de protection matérialisée figure 1. Cette forte interaction synergique a pour conséquence un pourcentage de protection non atteint pour les souris administrées avec les molécules seules, quelles qu'en soient les doses. Elle se traduit, dans le test d'alternance spontanée, en une amélioration des performances mnésiques des animaux intoxiqués (figure 1) traités avec la combinaison FENM-DPZ, en comparaison avec les animaux traités avec les molécules seules.

**2.2. Effet synergique d'une combinaison FENM-iChE sur la mémoire à long terme (PAT), dans un modèle d'intoxication à Aβ.**

Les données confirment les effets anti-amnésiques de la FENM et du DPZ sur les atteintes induites par les oligomères $A\beta_{25-35}$ sur la mémoire de travail à long terme.

Les souris intoxiquées à $A\beta_{25-35}$ et administrées avec les doses des 0,3 mg/Kg ou 1 mg/Kg de FENM IP montrent des performances non significativement différentes des souris témoins, non intoxiquées non traitées (figure 2) ; ces performances sont significativement différentes des souris non traitées intoxiquées. L'administration de la FENM à des doses plus faibles ne permet pas de protéger les souris contre les atteintes mnésiques dues à l'intoxication à $A\beta_{25-35}$. Seule la dose la plus importante de DPZ testée (1 mg/Kg) se montre efficace à contrer les effets amnésiants de l'intoxication aux oligomères $A\beta_{25-35}$ : le temps de latence des animaux est significativement différent de celui des animaux intoxiqués non traités. Néanmoins, l'amélioration ne suffit pas à restaurer complètement les performances des animaux puisque le temps de latence pour ceux-ci reste significativement différent du temps de latence des animaux non intoxiqués non traités.

L'administration combinée de FENM et de DPZ aux doses les plus faibles et inefficaces de FENM et de DPZ, respectivement de 0,01 et 0,03 mg/Kg, résulte en une protection significative des capacités de mémoire à long terme des souris intoxiquées, comparées à celles des souris intoxiquées non traitées (figure 2). De manière particulièrement intéressante ces doses sont inférieures de plus d'un ordre de grandeur que les première doses efficaces identifiées dans ce test, signant un effet de synergie particulièrement importante des composés sur la mémoire à long terme.

Comme indiqué dans le tableau 4 ci-dessous une telle protection à des doses de composés pourtant si faibles s'explique par une synergie particulièrement importante de chacun des composés, supportée par l'indice de combinaison particulièrement faible, qui signe un effet synergique particulièrement important.

Tableau 4

| traitement (mg/kg IP) | PP (%) | $C_{x,FENM}$ | $C_{x,DPZ}$ | CI |
|---|---|---|---|---|
| FENM (0) | $0,0 \pm 7,2$ | | | |

(suite)

| traitement (mg/kg IP) | PP (%) | $C_{x,FENM}$ | $C_{x,DPZ}$ | CI |
|---|---|---|---|---|
| FENM (0,01) | 1,4 ± 18,3 | | | |
| FENM (0,03) | -12,2 ± 16,8†† | | | |
| FENM (0,1) | 27,8 ± 24,8 | | | |
| FENM (0,3) | 52,9 ± 14,2 † | | | |
| DPZ (0) | 0,0 ± 7,2 | | | |
| DPZ (0,03) | 19,7 ± 17,2 | | | |
| DPZ (0,1) | 19,6 ± 21,6 | | | |
| DPZ (0,3) | 42,2 ± 21,4 ° | | | |
| FENM (0,01) + DPZ (0,03) | 84,9 ± 22,8 | 0,469 ± 0,076 | 0,563 ± 0,095 | **0,07 ± 0,01** |

PP : pourcentage de protection ; CI: indice de combinaison. $C_{x,FENM}$ et $C_{x,DPZ}$ ont été calculés à partir de la droite de régression des courbes dose-réponse de chacun des composés seuls : †y=180.98x, $R^2$ = 0,891; °y = 150,83x, $R^2$ = 0,898. ††un PP négatif résulte en un $C_{x,FENM}$ négatif.

### 3. Conclusion

Ces données montrent une synergie des effets protecteurs de la FENM combinés à ceux d'un iChE, tant pour la mémoire de travail à court terme que pour la mémoire de travail à long terme. En outre cette synergie est propre à l'utilisation de la FENM puisqu'elle n'est pas observée pour des combinaisons dans lesquelles la mémantine remplace la FENM.

Ces résultats permettent d'envisager l'utilisation de combinaisons FENM-iChE chez des patients actuellement sans solution thérapeutique approuvée pour le traitement des symptômes mnésiques de souffrant de MA ou de pathologies apparentées, tels que les patients au stade précoce de la maladie. Également, la synergie observée qui permet d'obtenir un effet thérapeutique à des doses particulièrement faibles des composés auxquelles peu voire aucun d'effet indésirable n'est attendu.

### REFERENCES

Chumakov I, Nabirotchkin S, Cholet N, Milet A, Boucard A, et al.. Combining two repurposed drugs as a promising approach for Alzheimer's disease therapy. Sci Rep. 2015 Jan 8;5:7608.

Couly S, Denus M, Bouchet M, Rubinstenn G, Maurice T. Anti-Amnesic and Neuroprotective Effects of Fluoroethylnormemantine in a Pharmacological Mouse Model of Alzheimer's Disease. Int J Neuropsychopharmacol. 2021;24:142-157.

Fraser TR. Lecture on the antagonism between the actions of active substances. Br Med J. 1872;2:485-7.

Kilkenny C, Browne W, Cuthill IC, Emerson M, Altman DG; NC3Rs Reporting Guidelines Working Group. Animal research: reporting in vivo experiments: the ARRIVE guidelines. Br J Pharmacol. 2010;160:1577-9.

Maekawa Y, Hasegawa S, Ishizuka T, Shiosakai K, Ishizuka H. Pharmacokinetics and Bioequivalence of Memantine Tablet and a New Dry Syrup Formulation in Healthy Japanese Males: Two Single-Dose Crossover Studies. Adv Ther. 2019 Oct;36(10):2930-2940.

Martin P, de Witte PAM, Maurice T, Gammaitoni A, Farfel G, Galer B. Fenfluramine acts as a positive modulator of sigma-1 receptors. Epilepsy Behav. 2020;105:106989.

Maurice T, Hiramatsu M, Itoh J, Kameyama T, Hasegawa T, Nabeshima T. Behavioral evidence for a modulating role of sigma ligands in memory processes. I. Attenuation of dizocilpine (MK-801)-induced amnesia. Brain Res. 1994a;647:44-56

Maurice T, Lockhart BP, Privat A (1996) Amnesia induced in mice by centrally administered β-amyloid peptides involves cholinergic dysfunction. Brain Res. 706:181-93.

Maurice T, Su TP, Parish DW, Nabeshima T, Privat A. PRE-084, a sigma selective PCP derivative, attenuates MK-801-induced impairment of learning in mice. Pharmacol Biochem Behav. 1994b;49:859-69.

Maurice T. Protection by sigma-1 receptor agonists is synergic with donepezil, but not with memantine, in a mouse model of amyloid-induced memory impairments. Behav Brain Res. 2016;296:270-278.

Meunier J, Ieni J, Maurice T. The anti-amnesic and neuroprotective effects of donepezil against amyloid beta25-35 peptide-induced toxicity in mice involve an interaction with the sigma1 receptor. Br J Pharmacol. 2006;149:998-1012.

Remington: The Science and Practice of Pharmacy (23rd ed.), Ed. A Adeboye Adejare, 2020.

Rogers SL, Friedhoff LT. Pharmacokinetic and pharmacodynamic profile of donepezil HCl following single oral doses. Br J Clin Pharmacol. 1998 Nov;46 Suppl 1(Suppl 1):1-6.

Rossetti HC, Munro Cullum C, Hynan LS, Lacritz LH. The CERAD Neuropsychologic Battery Total Score and the progression of Alzheimer disease. Alzheimer Dis Assoc Disord. 2010;24(2):138-142.

Stahl PH, Wermuth CG. Pharmaceutical Salts: Properties, Selection, and Use. Wiley, 2011. 388 pages.

Zhang, N., Zheng, X., Liu, H. et al. Testing whether the progression of Alzheimer's disease changes with the year of publication, additional design, and geographical area: a modeling analysis of literature aggregate data. Alz Res Therapy 12, 64 (2020).

## Revendications

1. Composition comprenant une combinaison synergique de 3-(2-fluoroéthyl)adamantan-1-amine (FENM) ou l'un quelconque de ses sels pharmaceutiquement acceptable et d'au moins un inhibiteur de l'acétylcholinestérase ou l'un quelconque de ses sels pharmaceutiquement acceptable.

2. La composition selon la revendication 1 dans laquelle ledit au moins un inhibiteur de l'acétylcholine estérase est sélectionné parmi le donépézil, la galantamine, la rivastigmine, la tacrine, ou l'un quelconque de leurs sels pharmaceutiquement acceptable.

3. La composition selon l'une quelconque des revendications 1 à 2, dans laquelle la dose de FENM est compatible avec un dosage de FENM inférieur ou égal à 20 mg/jour.

4. La composition selon l'une quelconque des revendications 1 à 3, dans laquelle ledit au moins un inhibiteur d'acétylcholinestérase est le donépézil qui est présent à une dose compatible avec un dosage inférieur ou égal à 10 mg/jour.

5. La composition selon l'une quelconque des revendications 1 à 4, dans laquelle ledit au moins un inhibiteur d'acétylcholinestérase est la rivastigmine qui est présente à une dose compatible avec un dosage inférieur ou égal à 3 mg/jour.

6. La composition selon l'une quelconque des revendications 1 à 5, dans laquelle ledit au moins un inhibiteur d'acétylcholinestérase est la galantamine qui est présente à une dose compatible avec un dosage inférieur ou égal à 16 mg/jour.

7. La composition selon l'une quelconque des revendications 1 à 6 dans laquelle le ratio molaire FENM/au moins un inhibiteur d'acétylcholine estérase est inférieur ou égal à 4, inférieur ou égal à 3, inférieur ou égal à 2, de préférence inférieur ou égal à 1.

8. Composition selon l'une quelconque des revendications 1 à 7 pour son utilisation en tant que médicament.

9. Composition selon l'une quelconque des revendications 1 à 8 pour son utilisation dans le traitement d'une pathologie sélectionnée parmi les tauopathies, synucléinopathies, les amyloïdopathies, la maladie d'Alzheimer, la maladie de Parkinson, l'atrophie multisystématisée, la maladie de Huntington, l'atrophie corticale postérieure, la maladie de Pick, l'épilepsie, la démence vasculaire, la démence fronto-temporale, la démence à corps de Lewy, la sclérose latérale amyotrophique.

10. La FENM, ou un sel pharmaceutiquement acceptable de celle-ci, en combinaison synergique avec au moins un inhibiteur de l'acétylcholinestérase sélectionné parmi le donépézil, la galantamine, la rivastigmine, la tacrine ou l'un quelconque de leurs sels pharmaceutiquement acceptable, pour une utilisation dans le traitement d'une pathologie sélectionnée parmi les tauopathies, synucléinopathies, les amyloïdopathies, la maladie d'Alzheimer, la maladie de Parkinson, l'atrophie multisystématisée, la maladie de Huntington, l'atrophie corticale postérieure, la maladie de Pick, l'épilepsie, la démence vasculaire, la démence fronto-temporale, la démence à corps de Lewy, la sclérose latérale amyotrophique.

**Patentansprüche**

1. Zusammensetzung, umfassend eine synergetische Kombination aus 3-(2-Fluorethyl)adamantan-1-amin (FENM) oder einem seiner pharmazeutisch akzeptablen Salze und mindestens einem Acetylcholinesterasehemmer oder einem seiner pharmazeutisch akzeptablen Salze.

2. Die Zusammensetzung nach Anspruch 1, wobei der mindestens eine Acetylcholinesterasehemmer aus Donepezil, Galantamin, Rivastigmin, Tacrin oder einem ihrer pharmazeutisch akzeptablen Salze ausgewählt ist.

3. Die Zusammensetzung nach einem der Ansprüche 1 bis 2, wobei die FENM-Dosis mit einer FENM-Dosierung von weniger als oder gleich 20 mg/Tag kompatibel ist.

4. Die Zusammensetzung nach einem der Ansprüche 1 bis 3, wobei der mindestens eine Acetylcholinesterasehemmer Donepezil ist, das in einer Dosis vorliegt, die mit einer Dosierung von weniger als oder gleich 10 mg/Tag kompatibel ist.

5. Die Zusammensetzung nach einem der Ansprüche 1 bis 4, wobei der mindestens eine Acetylcholinesterasehemmer Rivastigmin ist, das in einer Dosis vorliegt, die mit einer Dosierung von weniger als oder gleich 3 mg/Tag kompatibel ist.

6. Die Zusammensetzung nach einem der Ansprüche 1 bis 5, wobei der mindestens eine Acetylcholinesterasehemmer Galantamin ist, das in einer Dosis vorliegt, die mit einer Dosierung von weniger als oder gleich 16 mg/Tag kompatibel ist.

7. Die Zusammensetzung nach einem der Ansprüche 1 bis 6, wobei das Molverhältnis von FENM zu mindestens einem Acetylcholinesterasehemmer kleiner oder gleich 4, kleiner oder gleich 3, kleiner oder gleich 2, vorzugsweise kleiner oder gleich 1 ist.

8. Zusammensetzung nach einem der Ansprüche 1 bis 7 zu ihrer Verwendung als Arzneimittel.

9. Zusammensetzung nach einem der Ansprüche 1 bis 8 zu ihrer Verwendung bei der Behandlung einer Erkrankung, ausgewählt aus den Tauopathien, Synucleinopathien, den Amyloidopathien, der Alzheimer-Krankheit, der Parkinson-Krankheit, der multisystematischen Atrophie, der Huntington-Krankheit, der posterior kortikalen Atrophie, der Pick-Krankheit, der Epilepsie, der vaskulären Demenz, der frontotemporalen Demenz, der Lewy-Körper-Demenz, der amyotrophen Lateralsklerose.

10. Das FENM oder ein pharmazeutisch akzeptables Salz davon in synergetischer Kombination mit mindestens einem Acetylcholinesterasehemmer, ausgewählt aus Donepezil, Galantamin, Rivastigmin, Tacrin oder einem ihrer pharmazeutisch akzeptablen Salze, zur Verwendung bei der Behandlung einer Erkrankung, ausgewählt aus den Tauopathien, Synucleinopathien, den Amyloidopathien, der Alzheimer-Krankheit, der Parkinson-Krankheit, der multisystematischen Atrophie, der Huntington-Krankheit, der posterior kortikalen Atrophie, der Pick-Krankheit, der Epilepsie, der vaskulären Demenz, der frontotemporalen Demenz, der Lewy-Körper-Demenz, der amyotrophen Lateralsklerose.

**Claims**

1. A composition comprising a synergistic combination of 3-(2-fluoroethyl)adamantan-1-amine (FENM) or any one of its pharmaceutically-acceptable salts and at least one acetylcholinesterase inhibitor or any one of its pharmaceutically-acceptable salts.

2. The composition according to claim 1, wherein said at least one acetylcholine esterase inhibitor is selected from among donepezil, galantamine, rivastigmine, tacrine, or any one of their pharmaceutically-acceptable salts.

3. The composition according to any one of claims 1 to 2, wherein the FENM dose is compatible with a FENM dose less than or equal to 20 mg/day.

4. The composition according to any one of claims 1 to 3, wherein said at least one acetylcholinesterase inhibitor is donepezil which is present at a dose compatible with a dosage less than or equal to 10 mg/day.

5. The composition according to any one of claims 1 to 4, wherein said at least one acetylcholinesterase inhibitor is the rivastigmine which is present at a dose compatible with a dosage less than or equal to 3 mg/day.

6. The composition according to any one of claims 1 to 5, wherein said at least one acetylcholinesterase inhibitor is galantamine which is present at a dose compatible with a dosage less than or equal to 16 mg/day.

7. The composition according to any one of claims 1 to 6, wherein the FENM/at least one acetylcholine esterase inhibitor molar ratio is less than or equal to 4, less than or equal to 3, less than or equal to 2, preferably less than or equal to 1.

8. The composition according to any one of claims 1 to 7 for use thereof as a drug.

9. The composition according to any one of claims 1 to 8 for use thereof in the treatment of a pathology selected from among tauopathies, synucleinopathies, amyloidopathies, Alzheimer's disease, Parkinson's disease, multiple system atrophy, Huntington's disease, posterior cortical atrophy, Pick's disease, epilepsy, vascular dementia, frontotemporal dementia, dementia with Lewy bodies, amyotrophic lateral sclerosis.

10. FENM, or a pharmaceutically-acceptable salt thereof, in synergistic combination with at least one acetylcholinesterase inhibitor selected from among donepezil, galantamine, rivastigmine, tacrine, or any one of their pharmaceutically-acceptable salts, for use in the treatment of a pathology selected from among tauopathies, synucleinopathies, amyloidopathies, Alzheimer's disease, Parkinson's disease, multiple system atrophy, Huntington disease, posterior cortical atrophy, Pick's disease, epilepsy, vascular dementia, frontotemporal dementia, dementia with Lewy bodies, amyotrophic lateral sclerosis.

FIGURE 1

FIGURE 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2014191424 A
- WO 2019115833 A
- WO 2013064579 A

**Littérature non-brevet citée dans la description**

- *CHEMICAL ABSTRACTS*, 1639210-26-6
- *CHEMICAL ABSTRACTS*, 120014-06-4
- *CHEMICAL ABSTRACTS*, 120011-70-3
- *CHEMICAL ABSTRACTS*, 357-70-0
- *CHEMICAL ABSTRACTS*, 1953-04-4
- *CHEMICAL ABSTRACTS*, 5072-47-9
- *CHEMICAL ABSTRACTS*, 123441-03-2
- *CHEMICAL ABSTRACTS*, 129101-54-8
- *CHEMICAL ABSTRACTS*, 321-64-2
- *CHEMICAL ABSTRACTS*, 1684-40-8
- *CHEMICAL ABSTRACTS*, 7149-50-0
- Remington: The Science and Practice of Pharmacy. 2020
- **CHUMAKOV I** ; **NABIROTCHKIN S** ; **CHOLET N** ; **MILET A** ; **BOUCARD A et al.** Combining two repurposed drugs as a promising approach for Alzheimer's disease therapy.. *Sci Rep.*, 08 January 2015, vol. 5, 7608
- **COULY S** ; **DENUS M** ; **BOUCHET M** ; **RUBINS-TENN G** ; **MAURICE T**. Anti-Amnesic and Neuroprotective Effects of Fluoroethylnormemantine in a Pharmacological Mouse Model of Alzheimer's Disease.. *Int J Neuropsychopharmacol.*, 2021, vol. 24, 142-157
- **FRASER TR**. Lecture on the antagonism between the actions of active substances.. *Br Med J.*, 1872, vol. 2, 485-7
- **KILKENNY C** ; **BROWNE W** ; **CUTHILL IC** ; **EMERSON M** ; **ALTMAN DG**. NC3Rs Reporting Guidelines Working Group. Animal research: reporting in vivo experiments: the ARRIVE guidelines.. *Br J Pharmacol.*, 2010, vol. 160, 1577-9
- **MAEKAWA Y** ; **HASEGAWA S** ; **ISHIZUKA T** ; **SHIOSAKAI K** ; **ISHIZUKA H**. Pharmacokinetics and Bioequivalence of Memantine Tablet and a New Dry Syrup Formulation in Healthy Japanese Males: Two Single-Dose Crossover Studies.. *Adv Ther.*, October 2019, vol. 36 (10), 2930-2940
- **MARTIN P** ; **DE WITTE PAM** ; **MAURICE T** ; **GAMMAITONI A** ; **FARFEL G** ; **GALER B**. Fenfluramine acts as a positive modulator of sigma-1 receptors.. *Epilepsy Behav.*, 2020, vol. 105, 106989

- **MAURICE T** ; **HIRAMATSU M** ; **ITOH J** ; **KAMEYA-MA T** ; **HASEGAWA T** ; **NABESHIMA T**. Behavioral evidence for a modulating role of sigma ligands in memory processes. I. Attenuation of dizocilpine (MK-801)-induced amnesia.. *Brain Res.*, 1994, vol. 647, 44-56
- **MAURICE T** ; **LOCKHART BP** ; **PRIVAT A**. Amnesia induced in mice by centrally administered β-amyloid peptides involves cholinergic dysfunction.. *Brain Res.*, 1996, vol. 706, 181-93
- **MAURICE T** ; **SU TP** ; **PARISH DW** ; **NABESHIMA T** ; **PRIVAT A**. PRE-084, a sigma selective PCP derivative, attenuates MK-801-induced impairment of learning in mice.. *Pharmacol Biochem Behav.*, 1994, vol. 49, 859-69
- **MAURICE T**. Protection by sigma-1 receptor agonists is synergic with donepezil, but not with memantine, in a mouse model of amyloid-induced memory impairments.. *Behav Brain Res.*, 2016, vol. 296, 270-278
- **MEUNIER J** ; **LENI J** ; **MAURICE T**. The anti-amnesic and neuroprotective effects of donepezil against amyloid beta25-35 peptide-induced toxicity in mice involve an interaction with the sigma1 receptor.. *Br J Pharmacol.*, 2006, vol. 149, 998-1012
- **ROGERS SL** ; **FRIEDHOFF LT**. Pharmacokinetic and pharmacodynamic profile of donepezil HCl following single oral doses.. *Br J Clin Pharmacol.*, November 1998, vol. 46 (1), 1-6
- **ROSSETTI HC** ; **MUNRO CULLUM C** ; **HYNAN LS** ; **LACRITZ LH**. The CERAD Neuropsychologic Battery Total Score and the progression of Alzheimer disease.. *Alzheimer Dis Assoc Disord.*, 2010, vol. 24 (2), 138-142
- **STAHL PH** ; **WERMUTH CG**. Pharmaceutical Salts: Properties, Selection, and Use.. Wiley, 2011, 388
- **ZHANG, N.** ; **ZHENG, X.** ; **LIU, H. et al.** Testing whether the progression of Alzheimer's disease changes with the year of publication, additional design, and geographical area: a modeling analysis of literature aggregate data.. *Alz Res Therapy*, 2020, vol. 12, 64